# FASCICULE DE BREVET EUROPEEN

(11) **EP 3 691 446 B1**
(45) Date de publication et mention de la délivrance du brevet: **20.05.2026**
(21) Numéro de dépôt: 18799574.1
(22) Date de dépôt: 02.10.2018
(51) Int. Cl.: C12M 3/10, A01K 45/00

(54) **DISPOSITIF POUR INJECTION IN-OVO**
IN-OVO INJEKTOR
IN-OVO INJECTION DEVICE

(30) Priorité: 03.10.2017 FR 1759249
(43) Date de publication de la demande: 12.08.2020
(73) Titulaire: Nectra, 35740 Pacé (FR)
(72) Inventeur: ADJANOHOUN, Ephrem, 35740 Pacé (FR); YVIN, Jean Claude, 29250 Plougoulm (FR)
(74) Mandataire: Santarelli
(86) Numéro de dépôt international: PCT/FR2018/052426
(87) Numéro de publication internationale: WO 2019/069012

(56) Documents cités:
- US-A- 3 616 262
- US-A- 5 136 979
- US-A1- 2006 075 973

## Description

La présente invention est du domaine de l'injection de compositions dans les œufs à travers la coquille, également qualifiée d'injection in-ovo, et concerne en particulier un dispositif pour l'injection de compositions dans des œufs, et plus particulièrement les œufs d'oiseaux tels que les volatiles.

L'injection de compositions dans les œufs fécondés permet le traitement préventif ou curatif des embryons. Les compositions injectées sont, par exemple, des vaccins, des antibiotiques, des compléments alimentaires, des microorganismes et/ou des composants destinés à réduire la mortalité, améliorer la croissance embryonnaire, à produire des vaccins ou à faire des analyses. L'injection de compositions dans des œufs fécondés est toutefois exclue du périmètre de la présente invention. Une telle technique peut-être également utile pour l'injection de substances dans des œufs stériles, par exemple en vue de l'adjonction de conservateurs, ou pour la collecte d'échantillons d'œufs à travers la coquille etc.

De telles injections sont généralement réalisées au moyen de dispositifs de l'art antérieur comportant une tête d'injection positionnée de sorte à pénétrer dans les œufs à traiter par l'une de leurs extrémités.

Dans ce contexte, une approche connue décrite dans le brevet US 3,377,989, met en œuvre une machine pour l'injection automatisée in-ovo de matériel biologique qui comporte une aiguille fixe, portée par une enveloppe protectrice escamotable positionnée sur un support lui-même légèrement mobile, et destinée à percer la coquille et à injecter la solution in-ovo par mise en butée de l'œuf sur l'aiguille. L'inconvénient principal d'une telle technique, est, que bien que le support soit mobile et enchâssé dans un ressort pour amortir ses déplacements, il est difficile de contrôler la profondeur de pénétration de l'aiguille dans l'œuf ce qui occasionne des blessures irréversibles de l'embryon lorsque l'aiguille pénètre dans l'œuf pour injecter la solution in-ovo. Cette technique est également peu fiable car il arrive souvent que l'aiguille soit bouchée par un fragment de coquille lors du perçage, lequel fragment risque d'être entrainé en tout ou partie profondément à l'intérieur de l'œuf lors d'une nouvelle injection, provoquant ainsi une contamination potentielle des autres embryons. De plus, une telle technique ne permet pas de contrôler finement la vitesse d'impact de l'aiguille, et/ou du reste de la tête d'injection, sur la coquille ce qui occasionne de nombreuses pertes dues aux casses des œufs.

Il est également connu de l'art antérieur, et décrit dans le brevet US 3,616,262, un appareil pour l'automatisation des tâches effectuées lors du traitement des œufs fécondés, lequel comprend une aiguille coulissant dans un tube, et peut comprendre quatre ressorts hélicoïdaux permettant le retour vers une position de repos après infection dans l'œuf. Un tel appareil ne comprend toutefois aucun moyen de verrouillage apte à bloquer le déplacement du tube ou à maintenir l'aiguille dans le tube. Ainsi, un tel appareil ne permet également pas de contrôler précisément la profondeur de pénétration de l'aiguille ni la pression appliquée sur l'œuf.

Une autre approche développée, également connue de l'art antérieur et décrite dans la demande WO2007/024952, met en œuvre un système d'injection comportant une pompe reliée à une aiguille, laquelle aiguille est montée coulissante dans un perforateur (dont la traduction littérale anglaise : *« punch* ») destiné à perforer l'œuf, avant le coulissement de l'aiguille vers le centre de l'œuf. Une telle technique, bien qu'elle permette de réduire l'impact de l'outil d'injection et d'ôter les morceaux de coquille bloqués dans le perforateur, ne permet pas de contrôler ni la profondeur de pénétration du perforateur à l'intérieur de l'œuf, ni la profondeur de pénétration de l'aiguille d'injection. En effet, la taille des œufs étant variable, et les injecteurs fixés sur un support commun se translatant verticalement lors des phases de perçage et d'injection, le perforateur et l'aiguille d'injection pénètrent plus profondément dans les œufs de taille importante et moins profondément dans les œufs plus petits, le perforateur peut alors descendre jusqu'aux parties les plus sensibles de l'œuf, et, l'aiguille d'injection peut par suite présenter une course trop longue pouvant blesser l'embryon.

Il est également connu de l'art antérieur, et décrit dans la demande de brevet US 2006/075973, un appareil pour l'injection in-ovo comprenant un tube à double débattement incorporé dans une chambre dans lequel coulisse une aiguille, et pouvant comprendre des moyens de buté réglant la course de l'aiguille. Toutefois, les moyens de buté d'un tel appareil ne permettent ni de maintenir l'aiguille dans le tube, ni de retenir le mouvement du tube, si bien qu'un tel appareil ne permet pas non plus un contrôle précis de la profondeur de ponction de l'aiguille dans l'œuf.

Enfin, il est connu de l'art antérieur, et décrit dans le brevet US 5,136,979, un appareil d'injection comportant une pluralité d'injecteurs, comprenant chacun un ressort permettant que le trocart de l'injecteur perce l'œuf avant que l'aiguille de l'injecteur ne pénètre dans l'œuf, l'appareil comprenant également un ensemble de deux plaques permettant de stabiliser à la fois les œufs et l'injecteur. Un tel appareil d'injection ne comprend toutefois pas de moyens de verrouillage permettant d'empêcher les mouvements du trocart dans l'œuf, si bien que les inconvénients précités s'appliquent également à cet appareil.

Ainsi, la présente invention vise à résoudre les problèmes de l'art antérieur en proposant un dispositif pour l'injection d'une composition dans un œuf, tel que décrit dans la revendication 1, permettant de contrôler finement le débattement et la course du trocart et de l'aiguille, de manière à éviter de casser ou fêler la coquille.

Dans la suite de la présente description et dans le contexte de la présente invention, le terme « trocart » sera utilisé pour désigner un moyen de perforation de l'œuf dans lequel doit coulisser une aiguille d'injection.

La présente invention vise à résoudre les problèmes de l'art antérieur et concerne à cet effet, un dispositif pour l'injection d'une composition dans un œuf tel que décrit dans la revendication 1.

Le trocart est relié à l'enveloppe interne, c'est-à-dire que le trocart est soit fixé à l'enveloppe interne, soit le trocart coopère avec cette enveloppe dans des configurations de l'organe de guidage où le trocart est lié directement ou indirectement en appui sur l'enveloppe interne. Une extrémité du trocart prévu pour entrer en contact avec un œuf à traiter est découpée, par exemple sensiblement en biseau, pour permettre un perçage facilité de la coquille.

Le moyen d'injection est avantageusement une aiguille d'injection pour l'injection de substance liquide, qualifiée de solution, en pénétrant dans l'œuf à une profondeur maîtrisée. Le moyen d'injection est avantageusement monté coulissant au moins en partie dans ledit corps tubulaire du trocart entre une position où son extrémité d'injection est totalement rentrée dans le corps tubulaire, et une position où son extrémité d'injection fait saillie en dehors du corps tubulaire du trocart.

Le moyen d'injection peut alternativement être une buse qui permette l'injection d'au moins une substance gazeuse, et/ou la distribution d'au moins un corps solide qui doivent être soit propulsés à l'intérieur de l'œuf depuis l'extérieur via le corps tubulaire du trocart, soit introduits directement dans la zone d'intérêt à traiter dans l'œuf. Une telle buse peut fonctionner, par exemple à l'aide d'un gaz propulseur pour l'émission d'un jet.

Grâce à un tel dispositif le verrouillage et de déverrouillage asynchrone du trocart et de l'aiguille d'injection est possible. Le dispositif selon l'invention permet en outre de contrôler finement le débattement et la course du trocart et de l'aiguille au cours des étapes de perçage de la coquille et d'injection de la composition dans l'œuf. Durant la phase de perçage, la pression exercée par l'injecteur sur la coquille de l'œuf, ne s'applique uniquement que sur le trocart en verrouillant dans le même temps la position verticale de l'aiguille d'injection.

L'organe de guidage du dispositif selon l'invention comporte avantageusement :
- un premier rainurage ménagé sur la face externe de l'enveloppe interne et un deuxième rainurage ménagé sur la face interne de l'enveloppe externe, le premier et le deuxième rainurages s'étendent longitudinalement dans l'organe de guidage et sensiblement en vis-à-vis, le premier rainurage comprend le premier moyen de verrouillage comportant un premier coude positionné à l'une des extrémités du premier rainurage, le deuxième rainurage comprend le deuxième moyen de verrouillage comportant un coude, désigné deuxième coude, positionné à l'une de ses extrémités ; et
- un plot de liaison monté glissant à la fois dans le premier et le deuxième rainurage ;
le premier coude étant configuré pour un verrouillage du plot de liaison en étant libre en glissement dans le deuxième rainurage et apte à venir en appui sur une extrémité du deuxième rainurage ; et le deuxième coude étant configuré pour un blocage du plot de liaison en étant libre en glissement dans le premier rainurage et apte à venir en appui sur une extrémité du premier rainurage.

Le terme « coude » dans le cadre de l'invention désigne une encoche qui s'étend sur une extrémité de la partie linéaire de plus grande longueur d'un rainurage.

Un tel système à double rainurage permet un contrôle fin du déplacement des deux enveloppes dans l'organe de guidage, et assure un blocage avec amortissement grâce à une mise en butée progressive dans les coudes. Les deux rainurages sont sensiblement en vis-à-vis, ils ne sont pas nécessairement parfaitement alignés radialement par rapport à l'axe de l'organe de guidage, et peuvent être décalés l'un par rapport à l'autre. Les coudes sont positionnés de la manière suivante : en faisant coulisser l'enveloppe interne dans l'enveloppe externe l'évidement du premier coude s'étend radialement par rapport à l'axe de l'organe de guidage dans la lumière du deuxième rainurage. De même, en faisant coulisser l'enveloppe interne dans l'enveloppe externe l'évidement du deuxième coude s'étend radialement par rapport à l'axe de l'organe de guidage dans la lumière du premier rainurage. En positionnant le dispositif d'injection in-ovo verticalement, pointe de l'aiguille (lorsque le moyen d'injection est une aiguille) dirigée vers le bas : il devient possible de provoquer la descente du trocart en bloquant le déplacement du moyen d'injection, et à l'inverse, de provoquer la descente du moyen d'injection en bloquant le déplacement du trocart. Après que le trocart ait percé la coquille et pénétré à l'intérieur de l'œuf à une profondeur définie, la pression sur celui-ci disparaît, seule l'extrémité distale de l'injecteur applique une force sur la partie supérieure de l'œuf, ce qui a pour effet de verrouiller la position verticale du trocart dans l'œuf et de déverrouiller le moyen d'injection, permettant ainsi de limiter et définir la profondeur de pénétration du trocart à l'intérieur de l'œuf et permettant la descente du moyen d'injection dans l'œuf à faible vitesse.

Le premier rainurage est avantageusement traversant dans l'enveloppe interne et le plot de liaison est fixé, d'une part, par une extrémité sur le trocart et traverse, d'autre part, le premier rainurage en étant logé dans le deuxième rainurage par son autre extrémité. Le plot de liaison est monté sur le trocart et vient déboucher dans le deuxième rainurage de l'enveloppe externe de sorte à donner un point d'appui au trocart en lien avec les deux enveloppes pour un meilleur maintien. Ainsi, la course du trocart peut s'arrêter, tandis que le moyen d'injection, mobile dans le trocart, peut descendre à son tour jusqu'à ce que l'organe de guidage occupe une configuration dans laquelle les mouvements de l'enveloppe interne par rapport à l'enveloppe externe sont tous bloqués.

Le deuxième rainurage est avantageusement traversant et traversé par le plot de liaison lequel comporte une tête adaptée pour venir coopérer avec les bords externes du deuxième rainurage ; la tête du plot de liaison est, par exemple, une tête de vis biseautée.

Selon d'autres modes de réalisation, le deuxième moyen de verrouillage comporte avantageusement d'une part un premier aimant, positionné sur la face externe de l'enveloppe interne, et d'autre part, un deuxième aimant positionné sur la face interne de l'enveloppe externe, lesdits premier et deuxième aimant étant positionnés en regard dans l'organe de guidage dans une configuration de l'organe de guidage qualifiée de configuration de perçage. Ce système à double aimants permet un déplacement des enveloppes de l'organe de guidage l'une par rapport l'autre sans mouvements brusques ni à-coups. Tant que la coquille de l'œuf n'est pas percée avec pénétration du trocart dans l'œuf, le moyen d'injection est maintenu immobile en position rétractée. La force magnétique F1 s'exerçant entre les deux aimants est supérieure à la force F2 nécessaire pour percer la coquille de l'œuf, mais est inférieure à la force d'appui F3 de la coupelle lorsque celle-ci est en contact avec la coquille de l'œuf. Par conséquent, la translation de l'enveloppe externe par rapport à l'enveloppe interne est possible uniquement lorsque la coupelle vient s'appuyer sur la coquille de l'œuf et que le trocart a pénétré l'œuf de la profondeur désirée.

Avantageusement, le premier moyen de verrouillage du dispositif décrit ci-dessus est constitué par une liaison rigide du trocart sur la coupelle. La coupelle et le trocart peuvent par exemple être une seule pièce réalisée par moulage.

Avantageusement, le dispositif selon l'invention comprend un troisième moyen de verrouillage apte à bloquer le déplacement du moyen d'injection dans une configuration de l'organe de guidage pour laquelle le moyen d'injection fait saillie en dehors du trocart, qualifiée de configuration d'injection. Ainsi le dispositif selon l'invention permet non seulement de pouvoir contrôler la profondeur d'insertion du trocart et de pouvoir injecter le moyen d'injection, par exemple une aiguille dans l'œuf au moment opportun, mais également, de pouvoir contrôler étroitement la profondeur d'injection dans l'œuf.

Avantageusement, l'enveloppe externe comporte un fond et le troisième moyen de verrouillage comprend ledit fond, une bordure de l'enveloppe interne étant prévue pour venir en butée sur ledit fond dans la configuration d'injection. Ainsi dans la configuration de l'organe de guidage dans laquelle les mouvements de l'enveloppe interne par rapport à l'enveloppe externe sont tous bloqués, c'est-à-dire dans la configuration d'injection, la course du moyen d'injection dans l'œuf est contrôlée par la position du fond et/ou la longueur de l'enveloppe externe, voire de l'enveloppe interne qui doit venir en butée sur le fond.

Avantageusement, dans le dispositif selon les modes de réalisations décrits dans le cadre de l'invention comportant lesdits premiers et deuxièmes rainurages, le troisième moyen de verrouillage est l'extrémité du deuxième rainurage opposée à celle qui comporte le premier coude. Ainsi, le plot de liaison bloqué dans le premier coude coulisse dans le deuxième rainurage et termine sa course dans la susdite extrémité du deuxième rainurage pour bloquer la descente du moyen d'injection dans l'œuf (configuration d'injection). Il devient possible de régler finement la course de l'aiguille en fonction de la longueur du deuxième rainurage.

Pour tous les modes de réalisation décrit précédemment dans le cadre de l'invention, l'enveloppe interne et l'enveloppe externe sont avantageusement de forme sensiblement cylindrique. Il peut également être recouru à la mise en œuvre d'enveloppes de section carrée, triangulaire... Cependant la préférence est pour les sections circulaires plus faciles à réaliser ou à redimensionner après fabrication.

La présente invention concerne aussi un procédé d'injection de composition in-ovo dans un œuf stérile, mettant en œuvre des dispositifs tel que décrit dans le cadre de l'invention et comprenant les étapes :
- démarrer la mise en compression de l'organe de guidage qui occupe une première configuration dans laquelle le plot de liaison est logé dans le deuxième coude et le moyen d'injection est entièrement rétractée ;
- maintenir la compression et par suite le coulissement de l'enveloppe externe par rapport à l'enveloppe interne avec déplacement du plot de liaison dans le premier rainurage jusqu'à ce que la coupelle vienne se placer sur la partie supérieure d'un œuf stérile selon une deuxième configuration de l'organe de guidage ;
- continuer à comprimer l'organe de guidage pour induire la perforation de la coquille par le trocart ;
- maintenir la mise en compression de l'organe de guidage jusqu'à perforer l'œuf et jusqu'à ce que le plot de guidage soit bloqué dans le premier coude pour bloquer le déplacement du trocart ; et
- comprimer encore l'organe de guidage pour provoquer le glissement du plot de liaison dans le deuxième rainurage et la descente du moyen d'injection jusqu'à ce que le dispositif occupe la configuration d'injection.

Le déplacement du plot de liaison dans le premier rainurage aboutit en fin de course au coulissement du plot de liaison dans le premier coude. Les étapes successives de ce coulissement du plot de liaison dans le premier coude provoquent une mise en rotation du trocart. Cette rotation du trocart a pour effet de réduire l'appui transversal dirigé vers le bas du trocart qui a déjà percé l'œuf. Cette rotation du trocart relâche donc la mise en pression sur l'œuf, ce qui permet de ne pas casser ou fêler la coquille de l'œuf.

La présente invention concerne également un autre procédé d'injection de composition in-ovo dans un œuf stérile, mettant en œuvre d'autres dispositifs tel que décrit dans le cadre de l'invention et comprenant les étapes :
- positionner le trocart sur la partie supérieure d'un œuf stérile ;
- mettre en pression l'organe de guidage sur la partie supérieure de l'œuf qui occupe la configuration de perçage dans laquelle lesdits premier et deuxième aimant sont positionnés en regard dans l'organe de guidage jusqu'à perçage de l'œuf ;
- maintenir la compression jusqu'à la rupture de l'aimantation entre le premier aimant et le deuxième aimant, et par suite le coulissement de l'enveloppe externe par rapport à l'enveloppe interne, ce qui induit la descente du trocart dans l'œuf percé jusqu'à ce que la coupelle vienne se placer sur la partie supérieure dudit œuf ; et
- comprimer encore l'organe de guidage pour provoquer la descente du moyen d'injection jusqu'à ce que l'organe de guidage occupe la configuration d'injection.

La description détaillée qui suit présente des modes de réalisation de la présente invention, uniquement donnés à titre illustratif et qui ne doivent nullement être interprétés comme limitatifs, et leurs figures annexées parmi lesquelles :
- la figure 1 représente une vue de profil d'un dispositif d'injection selon l'invention, en transparence pour voir l'intérieur du dispositif ;
- la figure 2 représente une vue de profil d'un dispositif d'injection selon le mode de réalisation montré à la figure 1, dans une autre configuration : un éclaté a été représenté pour mieux visualiser l'intérieur du dispositif ;
- La figure 3 représente le même dispositif, en deux exemplaires, placé sur une plateforme d'injection au-dessus d'une ligne avec deux œufs ;
- la figure 4 représente une vue de profil d'un dispositif d'injection selon le mode de réalisation montré à la figure 1, dans une autre configuration ;
- la figure 5 représente une vue de profil d'un dispositif d'injection selon le mode de réalisation montré aux figures précédentes, dans une autre configuration ;
- la figure 6 représente une vue de profil d'un dispositif d'injection selon le mode de réalisation montré aux figures 1 à 3, dans une autre configuration ;

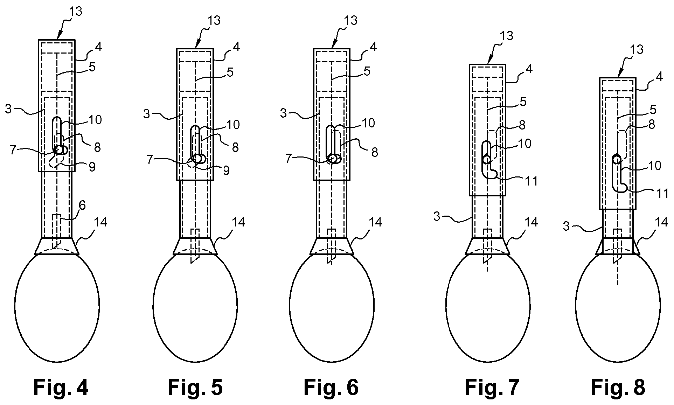

- la figure 5 représente une vue de profil d'un dispositif d'injection selon le mode de réalisation montré aux précédentes, dans une autre configuration ;
- la figure 7 représente une vue de profil d'un dispositif d'injection selon le mode de réalisation montré aux figures précédentes, dans une autre configuration ; et
- la figure 8 représente une vue de profil d'un dispositif d'injection selon le mode de réalisation montré aux figures précédentes, dans une autre configuration ; et

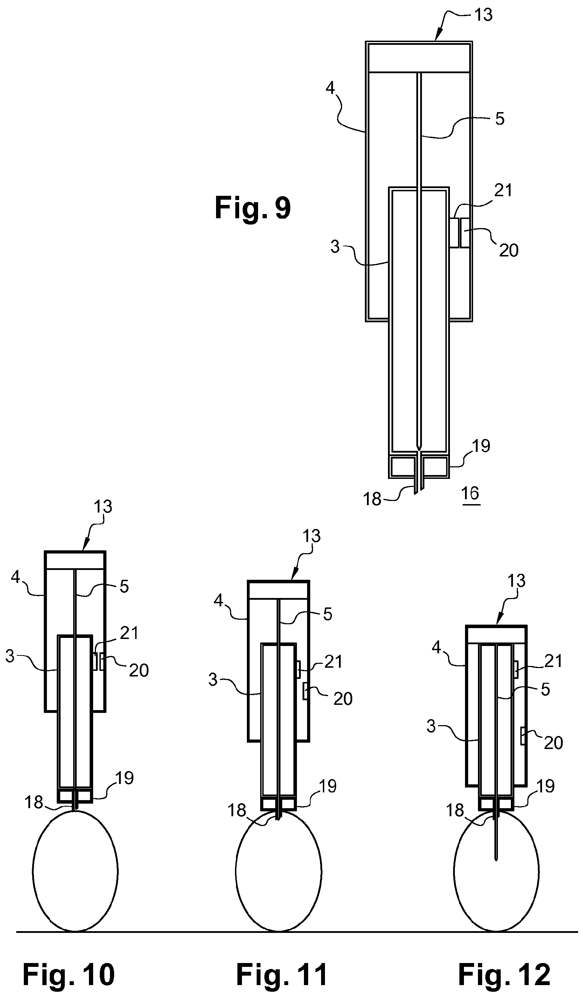

- la figure 9 représente une vue en coupe d'un autre mode de réalisation selon l'invention ;
- la figure 10 représente une vue en coupe du dispositif montré sur la figure 9 positionné sur un œuf ;
- la figure 11 représente une vue en coupe du dispositif montré sur la figure 10 dans une autre configuration ;
- la figure 12 représente une vue en coupe du dispositif montré sur les figures 10 et 11, dans une autre configuration ;

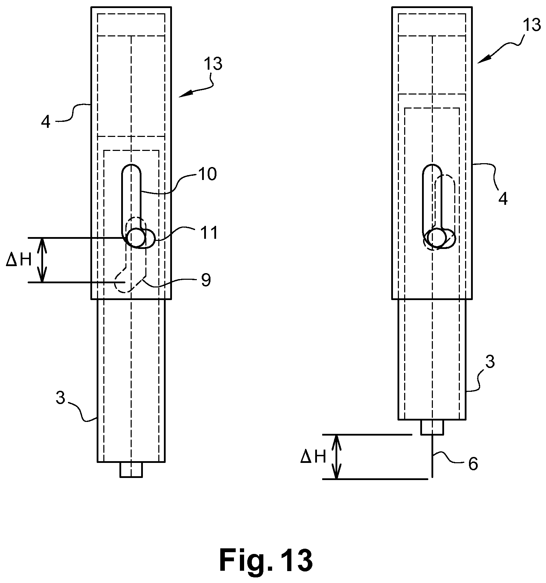

- la figure 13 montre l'organe de guidage et le trocart et les moyens de liaison du trocart à l'organe de guidage du dispositif selon l'invention suivant deux configurations extrêmes I et **II.**

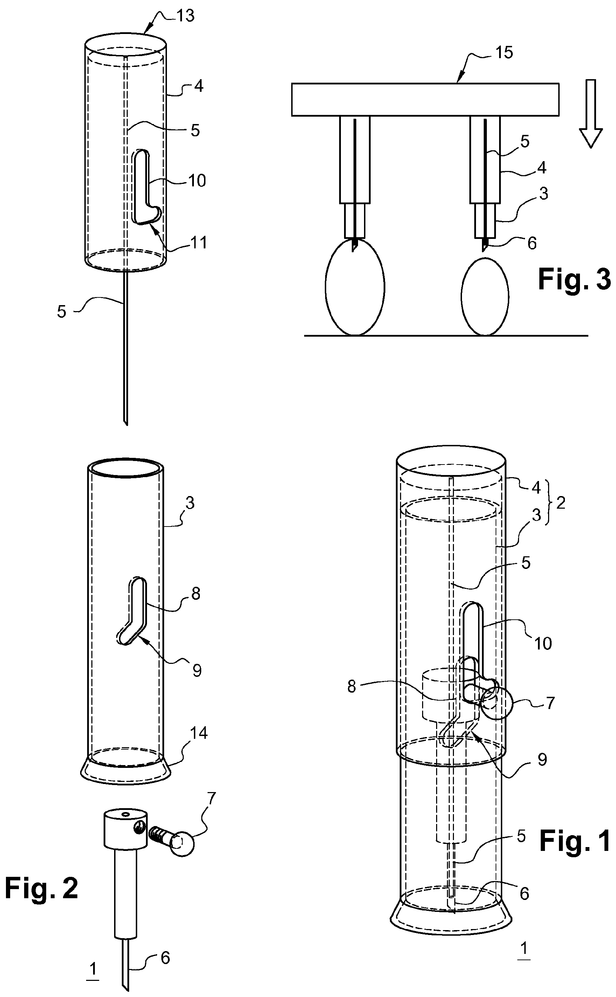

Sur les figures 1 et 2, est montré un premier mode de réalisation d'un dispositif pour l'injection in-ovo 1 selon l'invention, comportant un organe de guidage 2 composé d'une enveloppe interne 3 recouverte par une enveloppe externe 4, toutes deux de forme sensiblement cylindrique. L'enveloppe externe 4 est fixé à une aiguille d'injection 5 qui traverse un trocart 6 sur lequel est monté un plot de liaison 7 relié aux deux enveloppes, l'enveloppe interne 3 et l'enveloppe externe 4. Un rainurage, qualifié de premier rainurage 8, sillonne sur l'enveloppe interne 3 sur toute son épaisseur et comporte un coude, dénommé premier coude 9, qui s'étend sur une de ses extrémités. Un autre rainurage, qualifié de deuxième rainurage 10, sillonne sur l'enveloppe externe 4 sur toute son épaisseur et comporte un coude, dénommé deuxième coude 11, qui s'étend sur une de ses extrémités. Les deux coudes 9 et 11 sont positionnés du même côté - sur leur rainurage respectif - de l'organe de guidage 2.

Le plot de liaison 7 qui est monté sur le trocart 6 dans lequel coulisse l'aiguille d'injection 5, traverse les deux rainurages 8 et 10 et se termine par une tête biseautée qui vient glisser sur les bords du deuxième rainurage par la face externe de l'enveloppe externe, dits bords externes du deuxième rainurage.

Le premier coude 9 est configuré pour que sa lumière communique avec la lumière du deuxième rainurage 10, et, le premier coude 9 et le deuxième rainurage 10 sont tous les deux traversés par le plot de liaison 7 lors de la mise en mouvement de l'enveloppe interne 3 sur l'enveloppe interne 4 sur une première zone de débattement de l'organe de guidage 2. La première zone de débattement étant pour partie représentée aux figures 6 et 7. De même, le deuxième coude 11 est configuré pour que sa lumière communique avec la lumière du premier rainurage 8, et, le deuxième coude 11 et le premier rainurage 8 sont traversés par le plot de liaison 7 lors de la mise en mouvement de l'enveloppe interne 3 sur l'enveloppe interne 4 sur une deuxième zone de débattement de l'organe de guidage 2. La deuxième zone de débattement étant pour partie représentée au figures 1, 2, 3, 4 et 5.

L'enveloppe externe 4 comprend un fond 13 qui ferme une première extrémité de l'organe de guidage, et qui est fixé à une aiguille d'injection 5. L'enveloppe interne 3 comprend une coupelle 14 placée à la deuxième extrémité de l'organe de guidage, et conformée pour venir coiffer un œuf de poule.

Pour effectuer une injection d'un œuf de poule, le dispositif 1 selon l'invention monté sur une plateforme d'injection 15 est au repos dans une première configuration dans laquelle le plot de liaison 7 est logé dans le deuxième coude 11 et l'aiguille d'injection 5 est entièrement rétractée. Un œuf est amené au-dessous du dispositif 1 à l'aide d'un convoyeur et lorsqu'il est positionné au-dessous de la plateforme d'injection 15 en regard du dispositif 1, la plateforme 15 est abaissé pour que la coupelle 14 vienne coiffer la partie supérieure de l'œuf.

Pour effectuer l'injection d'une composition dans l'œuf par mise en compression de l'organe de guidage 2, lequel occupe au début une première configuration visible sur la figure 4, les étapes suivantes sont réalisées :
- placer un œuf sous l'organe de guidage 2 qui occupe (figure 4), au repos, une première configuration dans laquelle le plot de liaison 7 est logé dans le deuxième coude 11 et l'aiguille 5 est entièrement rétractée ;
- venir coiffer la partie supérieure de l'œuf avec la coupelle 14 ;
- une fois le contact établi, appliquer une compression de l'organe de guidage 2 sur l'œuf de sorte à entraîner le coulissement de l'enveloppe externe 4 par rapport à l'enveloppe interne 3 avec déplacement du plot de liaison 7 dans le premier rainurage 8 (voir figure 5) ;
- maintenir la mise en compression de l'organe de guidage 2 jusqu'à perforer l'œuf et jusqu'à ce que le plot de guidage 7 soit bloqué (voir figure 6) dans le premier coude 9 pour bloquer le déplacement du trocart 6 ; et
- continuer la compression pour induire le glissement du plot de liaison 7 dans le deuxième rainurage 10 et par suite, la descente de l'aiguille 5 (voir figure 7) jusqu'à ce que l'organe de guidage 2 soit dans la configuration d'injection (voir figure 8).

Le déplacement du plot de liaison 7 dans le premier rainurage 8 aboutit en fin de course au coulissement du plot de liaison 7 dans le premier coude 9. Les étapes successives de ce coulissement du plot de liaison 7 dans le premier coude 9 sont montrées de la figure 5 à la figure 7, ce qui provoque une mise en rotation du trocart 6. Cette rotation du trocart 6 a pour effet de réduire l'appui transversal dirigé vers le bas du trocart qui a déjà percé l'œuf. Cette rotation du trocart 6 relâche donc la mise en pression sur l'œuf, ce qui permet de ne pas casser ou fêler la coquille de l'œuf.

La figure 13 montre comment un tel dispositif permet de définir étroitement la profondeur de pénétration du trocart aux fins d'obvier les inconvénients énumérés ci-avant. La hauteur ΔH entre le premier coude 9 du premier rainurages 8 et le deuxième coude 11 du deuxième rainurage 10 selon l'axe longitudinal, et lorsque l'injecteur est dans la configuration de repos (configuration I) correspond à la profondeur maximale de pénétration du trocart à l'intérieur de l'œuf : c'est-à-dire à la distance maximale entre sensiblement la projection des rebords de la coupelle 14 sur l'axe du trocart (ici c'est l'extrémité basse de l'organe de guidage qui est montrée) et l'extrémité du trocart 6, lorsque l'injecteur est dans une configuration qualifiée de configuration de perçage (configuration II), cette configuration de perçage étant également représentée sur la figure 6.

Un autre mode de réalisation de dispositif pour l'injection in-ovo selon l'invention est représenté, le dispositif 16 sur la figure 9. Tout comme le dispositif 1 du mode de réalisation décrit précédemment dans le cadre de l'invention, ce dispositif 16 comporte un organe de guidage 17 composé d'une enveloppe interne 3 recouverte par une enveloppe externe 4, toutes deux de forme sensiblement cylindrique. L'enveloppe externe 4 comprend un fond 13 qui ferme une première extrémité de l'organe de guidage, et qui est fixé à une aiguille d'injection 5 qui traverse un trocart 18. Le trocart 18 est fixé sur une coupelle 19 placée à la deuxième extrémité de l'organe de guidage, et conformée pour venir coiffer un œuf de poule. Un premier aimant 20 est fixé sur la face interne de l'enveloppe externe 4, et en vis-à-vis d'un deuxième aimant 21 lui-même fixé sur la face externe de l'enveloppe interne 3 dans une configuration de l'organe de guidage qualifiée de configuration de perçage, représentée aux figures 9 et 10.

Pour effectuer l'injection d'une composition dans l'œuf par mise en compression de l'organe de guidage 2, lequel occupe au début la configuration de perçage visible sur les figures 9 ou 10, les étapes suivantes sont réalisées :
- positionner le trocart 18 sur la partie supérieure d'un œuf ;
- mettre en pression l'organe de guidage 2 sur la partie supérieure de l'œuf qui occupe la configuration de perçage dans laquelle lesdits premier et deuxième aimants 20, 21 sont positionnés en regard dans l'organe de guidage jusqu'à perçage de l'œuf ;
- maintenir la compression jusqu'à la rupture de l'aimantation entre le premier aimant et le deuxième aimant (figure 11), et par suite le coulissement de l'enveloppe externe par rapport à l'enveloppe interne, ce qui induit la descente du trocart dans l'œuf percé jusqu'à ce que la coupelle vienne se placer sur la partie supérieure dudit œuf ; et
- comprimer encore l'organe de guidage pour provoquer la descente de l'aiguille jusqu'à ce que l'organe de guidage occupe la configuration d'injection (figure 12).

## Revendications

1. Dispositif (1) pour l'injection d'une composition dans un œuf, comportant un trocart (6, 18) comprenant un corps tubulaire et un moyen d'injection (5) monté coulissant au moins en partie dans ledit corps tubulaire du trocart entre une position où l'extrémité d'injection du moyen d'injection est totalement rentrée dans le corps tubulaire, et une position où l'extrémité d'injection du moyen d'injection fait saillie en dehors du corps tubulaire du trocart, le dispositif comprenant en outre un premier moyen de verrouillage apte à bloquer le déplacement du trocart (6), un deuxième moyen de verrouillage apte à bloquer le déplacement du moyen d'injection (5) dans des configurations pour lesquelles ledit moyen d'injection est rentré dans le trocart (6), et un organe de guidage comprenant une première enveloppe et une deuxième enveloppe, ladite deuxième enveloppe étant reliée au trocart (6, 18) et comportant une coupelle (14) destinée à venir en appui sur la partie supérieure d'un œuf, **caractérisé en ce que** ladite première enveloppe est une enveloppe externe (4) fixée au moyen d'injection (5) et ladite deuxième enveloppe est une enveloppe interne (3) mobile en translation dans l'enveloppe externe (4), ledit organe de guidage (2) comprenant :
- un premier rainurage (8) ménagé sur la face externe de l'enveloppe interne (3) et un deuxième rainurage (10) ménagé sur la face interne de l'enveloppe externe (4), le premier et le deuxième rainurage (8 et 10) s'étendant longitudinalement dans l'organe de guidage (2) et sensiblement en vis-à-vis, le premier rainurage (8) comprenant le premier moyen de verrouillage comportant un premier coude (9) positionné à l'une des extrémités du premier rainurage, le deuxième rainurage comprenant le deuxième moyen de verrouillage comportant un coude, désigné deuxième coude (11), positionné à l'une de ses extrémités ; et
- un plot de liaison (7) monté glissant à la fois dans le premier et le deuxième rainurage ;
le premier coude (9) étant configuré pour un blocage du plot de liaison (7) libre en glissement dans le deuxième rainurage (10) et apte à venir en appui sur une extrémité du deuxième rainurage (10) ; et le deuxième coude (11) étant configuré pour un blocage du plot de liaison (7) libre en glissement dans le premier rainurage (8) et apte à venir en appui sur une extrémité du premier rainurage (8).

2. Dispositif selon la revendication 1, dans lequel le premier rainurage (8) est traversant dans l'enveloppe interne (3) et le plot de liaison (7) est fixé, d'une part, par une extrémité sur le trocart (6) et traverse, d'autre part, le premier rainurage (8) en étant logé dans le deuxième rainurage (10) par son autre extrémité.

3. Dispositif selon la revendication 2, dans lequel le deuxième rainurage (10) est traversant et traversé par le plot de liaison (7), lequel comporte une tête adaptée pour venir coopérer avec les bords externes du deuxième rainurage (10).

4. Dispositif selon la revendication 1, dans lequel le deuxième moyen de verrouillage comporte d'une part un premier aimant (20), positionné sur la face externe de l'enveloppe interne (3), et d'autre part, un deuxième aimant (21) positionné sur la face interne de l'enveloppe externe (4), lesdits premier et deuxième aimants (20 et 21) étant positionnés en regard dans l'organe de guidage (2) dans une configuration de l'organe de guidage qualifiée de configuration de perçage.

5. Dispositif selon la revendication 4, dans lequel le premier moyen de verrouillage est constitué par une liaison rigide du trocart (18) sur la coupelle (14).

6. Dispositif selon l'une des revendications 1 à 5, comprenant un troisième moyen de verrouillage apte à bloquer le déplacement du moyen d'injection (5) dans une configuration de l'organe de guidage pour laquelle ledit moyen d'injection fait saillie en dehors du trocart, dite configuration d'injection.

7. Dispositif selon la revendication 6, dans lequel l'enveloppe externe (4) comporte un fond (13) et le troisième moyen de verrouillage comprend ledit fond (13), une bordure de l'enveloppe interne (3) étant prévue pour venir en butée sur ledit fond (13) dans la configuration d'injection.

8. Dispositif selon les revendications 1 à 3, prises ensemble avec la revendication 6, dans lequel le troisième moyen de verrouillage est l'extrémité du deuxième rainurage (10) opposée à celle qui comporte le premier coude (9).

9. Dispositif (1) selon l'une des revendications 1 à 8, pour l'injection d'une solution liquide, dans lequel le moyen d'injection est une aiguille d'injection (5).

10. Procédé d'injection dans un œuf stérile mettant en œuvre le dispositif (1) selon l'une des revendications 1 à 3, éventuellement prise ensemble avec l'une des revendications 6 à 8, comprenant les étapes suivantes :
- placer un œuf stérile sous l'organe de guidage (2) qui occupe une première configuration dans laquelle le plot de liaison (7) est logé dans le deuxième coude (11) et le moyen d'injection (5) est entièrement rétractée ;
- maintenir la compression et par suite le coulissement de l'enveloppe externe (4) par rapport à l'enveloppe interne (3) avec déplacement du plot de liaison (7) dans le premier rainurage (3) jusqu'à ce que la coupelle (14) vienne se placer sur la partie supérieure d'un œuf selon une deuxième configuration de l'organe de guidage (2) ;
- continuer à comprimer l'organe de guidage (2) pour induire la perforation de la coquille par le trocart (6) ;
- maintenir la mise en compression de l'organe de guidage (2) jusqu'à perforer l'œuf et jusqu'à ce que le plot de liaison (7) soit bloqué dans le premier coude (9) pour bloquer le déplacement du trocart (6) ; et
- comprimer encore l'organe de guidage (2) pour provoquer le glissement du plot de liaison (7) dans le deuxième rainurage (10) et la descente du moyen d'injection (5) jusqu'à ce que le dispositif soit dans la configuration d'injection.

11. Procédé d'injection dans un œuf stérile mettant en œuvre le dispositif selon l'une des revendications 4 à 5, éventuellement prise ensemble avec l'une des revendications 6 ou 7, comprenant les étapes suivantes :
- positionner le trocart (18) sur la partie supérieure d'un œuf stérile ;
- mettre en pression l'organe de guidage (2) sur la partie supérieure de l'œuf qui occupe la configuration de perçage dans laquelle lesdits premier et deuxième aimants (20 et 21) sont positionnés en regard dans l'organe de guidage (2) jusqu'à perçage de l'œuf ;
- maintenir la compression jusqu'à la rupture de l'aimantation entre le premier aimant et le deuxième aimant (20 et 21), et par suite le coulissement de l'enveloppe externe (4) par rapport à l'enveloppe interne (3), ce qui induit la descente du trocart (18) dans l'œuf percé jusqu'à ce que la coupelle (19) vienne se placer sur la partie supérieure dudit œuf ; et
- comprimer encore l'organe de guidage (2) pour provoquer la descente du moyen d'injection (5) jusqu'à ce que le dispositif soit dans la configuration d'injection.

## Patentansprüche

1. Vorrichtung (1) zum Injizieren einer Zusammensetzung in ein Ei, umfassend einen Trokar (6, 18), der einen röhrenförmigen Körper und ein Injektionsmittel (5) umfasst, das mindestens teilweise in den röhrenförmigen Körper des Trokars gleitend zwischen einer Position, in der das Injektionsende des Injektionsmittels vollständig in den röhrenförmigen Körper zurückgezogen ist, und einer Position, in der das Injektionsende des Injektionsmittels aus dem röhrenförmigen Körper des Trokars herausragt, montiert ist, wobei die Vorrichtung ferner ein erstes Verriegelungsmittel umfasst, das geeignet ist, die Bewegung des Trokars (6) zu blockieren, ein zweites Verriegelungsmittel, das geeignet ist, die Bewegung des Injektionsmittels (5) in Konfigurationen zu blockieren, bei denen das Injektionsmittel in den Trokar (6) zurückgezogen ist, und ein Führungselement, das eine erste Hülle und eine zweite Hülle umfasst, wobei die zweite Hülle mit dem Trokar (6, 18) verbunden ist und eine Schale (14) aufweist, die dazu bestimmt ist, auf dem oberen Teil eines Eies aufzuliegen, **dadurch gekennzeichnet, dass** die erste Hülle eine äußere Hülle (4) ist, die an dem Injektionsmittel (5) befestigt ist, und die zweite Hülle eine innere Hülle (3) ist, die sich in der äußeren Hülle (4) verschieben lässt, wobei das Führungselement (2) Folgendes umfasst:
- eine erste Nut (8), die auf der Außenseite der inneren Hülle (3) vorgesehen ist, und eine zweite Nut (10), die auf der Innenseite der äußeren Hülle (4) vorgesehen ist, wobei sich die erste und die zweite Nut (8 und 10) längs in dem Führungselement (2) erstrecken und im Wesentlichen einander gegenüberliegen, wobei die erste Nut (8) das erste Verriegelungsmittel umfasst, das einen ersten Knick (9) umfasst, der an einem der Enden der ersten Nut positioniert ist, und die zweite Nut das zweite Verriegelungsmittel umfasst, das einen Knick, bezeichnet als zweiten Knick (11), umfasst, der an einem seiner Enden positioniert ist; und
- einen Verbindungsstift (7), der sowohl in der ersten als auch in der zweiten Nut gleitend montiert ist;
wobei der erste Knick (9) für eine Verriegelung des frei gleitenden Verbindungsstifts (7) in der zweiten Nut (10) und zu der Auflage an einem Ende der zweiten Nut (10) geeignet ist; und der zweite Knick (11) für eine Blockierung des frei gleitenden Verbindungsstifts (7) in der ersten Nut (8) und zu der Auflage an einem Ende der ersten Nut (8) geeignet ist.

2. Vorrichtung nach Anspruch 1, wobei die erste Nut (8) in der inneren Hülle (3) durchgehend ist und der Verbindungsstift (7) einerseits durch ein Ende an dem Trokar (6) befestigt ist und andererseits die erste Nut (8) durchquert, indem er durch sein anderes Ende in der zweiten Nut (10) gelagert ist.

3. Vorrichtung nach Anspruch 2, wobei die zweite Nut (10) durchgehend ist und von dem Verbindungsstift (7) durchquert wird, der einen Kopf umfasst, der geeignet ist, mit den Außenkanten der zweiten Nut (10) zusammenzuarbeiten.

4. Vorrichtung nach Anspruch 1, wobei das zweite Verriegelungsmittel einerseits einen ersten Magneten (20) aufweist, der auf der Außenseite der Innenhülle (3) positioniert ist, und andererseits einen zweiten Magneten (21), der auf der Innenseite der Außenhülle (4) positioniert ist, wobei der erste und der zweite Magnet (20 und 21) gegenüberliegend in dem Führungselement (2) in einer Konfiguration des Führungselements positioniert sind, das als Bohrkonfiguration qualifiziert ist.

5. Vorrichtung nach Anspruch 4, wobei das erste Verriegelungsmittel aus einer starren Verbindung des Trokars (18) mit der Schale (14) besteht.

6. Vorrichtung nach einem der Ansprüche 1 bis 5, umfassend ein drittes Verriegelungsmittel, das geeignet ist, die Bewegung des Injektionsmittels (5) in einer Konfiguration des Führungselements zu blockieren, bei der das Injektionsmittel aus dem Trokar herausragt, wobei diese Konfiguration als Injektionskonfiguration bezeichnet wird.

7. Vorrichtung nach Anspruch 6, wobei die äußere Hülle (4) einen Boden (13) umfasst und das dritte Verriegelungsmittel den Boden (13) umfasst, wobei ein Rand der inneren Hülle (3) vorgesehen ist, um in der Injektionskonfiguration an dem Boden (13) anzustoßen.

8. Vorrichtung nach den Ansprüchen 1 bis 3, zusammen mit Anspruch 6, wobei das dritte Verriegelungsmittel das Ende der zweiten Nut (10) gegenüber dem Ende ist, das den ersten Knick (9) umfasst.

9. Vorrichtung (1) nach einem der Ansprüche 1 bis 8 zum Injizieren einer flüssigen Lösung, wobei das Injektionsmittel eine Injektionsnadel (5) ist.

10. Verfahren zur Injektion in ein steriles Ei unter Verwendung der Vorrichtung (1) nach einem der Ansprüche 1 bis 3, gegebenenfalls zusammen mit einem der Ansprüche 6 bis 8, umfassend die folgenden Schritte:
- Platzieren eines sterilen Eies unter das Führungselement (2), das eine erste Konfiguration einnimmt, in der der Verbindungsstift (7) in dem zweiten Knick (11) untergebracht ist und das Injektionsmittel (5) vollständig eingezogen ist;
- Aufrechterhalten der Kompression und infolgedessen das Verschieben der äußeren Hülle (4) in Bezug auf die innere Hülle (3) mit Verschiebung des Verbindungsstifts (7) in der ersten Nut (3), bis sich die Schale (14) gemäß einer zweiten Konfiguration des Führungselements (2) auf dem oberen Teil eines Eies befindet;
- Fortführen des Komprimierens des Führungselements (2), um die Perforation der Schale durch den Trokar (6) herbeizuführen;
- Aufrechterhalten der Kompression auf das Führungselement (2), bis das Ei perforiert ist und bis der Verbindungsstift (7) in dem ersten Knick (9) blockiert ist, um die Bewegung des Trokars (6) zu blockieren; und
- weiteres Komprimieren des Führungselements (2), um das Gleiten des Verbindungsstifts (7) in der zweiten Nut (10) und das Absenken des Injektionsmittels (5) zu bewirken, bis sich die Vorrichtung in der Injektionskonfiguration befindet.

11. Verfahren zur Injektion in ein steriles Ei unter Verwendung der Vorrichtung nach einem der Ansprüche 4 bis 5, gegebenenfalls zusammen mit einem der Ansprüche 6 oder 7, umfassend die folgenden Schritte:
- Positionieren des Trokars (18) auf den oberen Teil eines sterilen Eies;
- Drücken des Führungselements (2) auf den oberen Teil des Eies, der die Bohrkonfiguration einnimmt, in der der erste und der zweite Magnet (20 und 21) gegenüberliegend in dem Führungselement (2) positioniert sind, bis das Ei durchbohrt ist;
- Aufrechterhalten der Kompression, bis die Magnetisierung zwischen dem ersten Magneten und dem zweiten Magneten (20 und 21) gebrochen ist, und infolgedessen das Verschieben der äußeren Hülle (4) in Bezug auf die innere Hülle (3), was das Absenken des Trokars (18) in das durchbohrte Ei bewirkt, bis sich die Schale (19) auf dem oberen Teil des Eies befindet; und
- weiteres Komprimieren des Führungselements (2), um das Absenken des Injektionsmittels (5) zu bewirken, bis sich die Vorrichtung in der Injektionskonfiguration befindet.

## Claims

1. Device (1) for injecting a composition into an egg, including a trocar (6, 18) comprising a tubular body and an injection means mounted such that it can slide at least partially inside said tubular body of the trocar (5) between a position in which the injection end of the injection means is fully retracted inside the tubular body, and a position in which the injection end of the injection means projects outside of the tubular body of the trocar, the device further comprising a first locking means suitable for blocking the movement of the trocar (6), a second locking means suitable for blocking the movement of the injection means (5) in configurations in which said injection means is retracted inside the trocar (6), and a guide member comprising a first casing and a second casing, said second casing being connected to the trocar (6, 18) and having a cup (14) intended to bear on the top part of an egg, **characterized in that** said first casing is an outer casing (4) secured to the injection means (5) and said second casing is an inner casing (3) which can move in translation inside the outer casing (4), said guide member (2) comprising:
- a first grooving (8) made on the outside face of the inner casing (3) and a second grooving (10) made on the inside face of the outer casing (4), the first and the second groovings (8 and 10) extending longitudinally in the guide member (2) and substantially facing one another, the first grooving (8) comprises the first locking means including a first bend (9) positioned at one of the ends of the first grooving, the second grooving comprises the second locking means having a bend, referred to as a second bend (11), positioned at one of the ends thereof; and
- a bonding stud (7) mounted such that it slides both inside the first and the second grooving; The first bend (9) being configured for blocking the bonding stud (7) free to slide inside the second grooving (10) and suitable of bearing against one end of the second grooving (10); and the second bend (11) being configured for blocking the bonding stud (7) free to slide inside the first grooving (8) and suitable of bearing against one end of the first grooving (8).

2. Device according to claim 1, wherein the first grooving (8) passes through the inner casing (3) and the bonding stud (7) is fastened, on the one hand, by one end to the trocar (6) and passes through, on the other hand, the first grooving (8) while being housed inside the second grooving (10) via the other end thereof.

3. Device according to claim 2, wherein the second grooving (10) is penetrating and is passed through by the bonding stud (7), which includes a head adapted for engaging with the outer edges of the second grooving (10).

4. Device according to claim 1, wherein said wherein the second locking means includes, on the one hand, a first magnet (20) positioned on the outside face of the inner casing (3), and on the other hand, a second magnet (21) positioned on the inside face of the outer casing (4), said first and second magnets (20 and 21) being positioned facing one another in the guide member (2) in a configuration of the guide member known as a puncturing configuration.

5. Device according to claim 4, wherein the first locking means is constituted by a rigid connection of the trocar (18) to the cup (14).

6. Device according to any of claims 1 to 5, comprising a third locking means suitable of blocking the displacement of the injection means (5) in a configuration of the guide member for which said injection means projects outside of the trocar, referred to as an injection configuration.

7. Device according to claim 6, wherein the outer casing (4) includes a bottom (13) and the third locking means comprises said bottom (13), a border of the inner casing (3) being provided so as to abut against said bottom (13) in the injection configuration.

8. Device according to claims 1 to 3, taken in combination with claim 6, wherein the third locking means is the end of the second grooving (10) opposite that which includes the first bend (9).

9. Device (1) according to claims 1 to 8, 9 for the injection of a liquid solution, wherein the injection means is an injection needle (5).

10. Injection process in a sterile egg implementing the device (1) according to one of claims 1 to 3, eventually taken in combination with one of claims 6 to 8, comprising the following steps:
- placing a sterile egg beneath the guide member (2) which occupies a first configuration wherein the bonding stud (7) is housed inside the second bend (11) and the injection means (5) is fully retracted;
- maintaining compression and subsequently the sliding of the outer casing (4) relative to the inner casing (3) with displacement of the bonding stud (7) in the first grooving (3) until the cup (14) becomes positioned on the top part of an egg according to a second configuration of the guide member (2);
- continuing to compress the guide member (2) in order to puncture the shell by the trocar (7);
- maintaining the compression setting of the guide member (2) until the egg is punctured and until the bonding stud (7) is blocked in the first bend (9) in order to block the displacement of the trocar (6); and
- further compressing the guide member (2) to cause the bonding stud (7) to slide inside the second grooving (10) and the injection means (5) to be lowered until the device is in the injection configuration.

11. Injection process in a sterile egg implementing the device (1) according to one of claim 4 or 5, eventually taken in combination with one of claims 6 or 7, comprising the following steps:
- positioning the trocar (18) on the top part of a sterile egg;
- pressurising the guide member (2) on the top part of the egg which occupies the puncturing configuration wherein said first and second magnets (20 and 21) are positioned facing one another in the guide member (2) until the egg is punctured;
- maintaining compression until the magnetisation between the first magnet and the second magnet (20 and 21) is broken, and subsequently until the sliding of the outer casing (4) relative to the inner casing (3), which results in the lowering of the trocar (18) into the punctured egg until the cup (19) is positioned on the top part of said egg; and
- further compressing the guide member (2) to cause the lowering of the injection means (5) until the device is in the injection configuration.
